# EUROPÄISCHE PATENTSCHRIFT

(11) **EP 0 880 346 B1**
(45) Veröffentlichungstag und Bekanntmachung des Hinweises auf die Patenterteilung: **12.05.2004**
(21) Anmeldenummer: 97901570.8
(22) Anmeldetag: 21.01.1997
(51) Int. Cl.: A61F 5/01

(54) **KNIEGELENKORTHESE**
KNEE JOINT ORTHESIS
ORTHESE DU GENOU

(30) Priorität: 16.02.1996 DE 19605734
(43) Veröffentlichungstag der Anmeldung: 02.12.1998
(73) Patentinhaber: Beiersdorf Aktiengesellschaft, 20245 Hamburg (DE)
(72) Erfinder: ALBROD, Andreas, D-21217 Seevetal (DE); HERZBERG, Thorsten, D-21149 Hamburg (DE)
(74) Vertreter: Dinné, Erlend
(86) Internationale Anmeldenummer: PCT/EP1997/000264
(87) Internationale Veröffentlichungsnummer: WO 1997/029718

(56) Entgegenhaltungen:
- DE-A- 4 309 577
- GB-A- 1 534 434
- US-A- 4 966 133
- US-A- 5 443 444

## Beschreibung

Die Erfindung betrifft eine Kniegelenkorthese, die zwei unterschiedliche Orthesengelenke aufweist, so daß eine Verlagerung der Drehachse der Kniegelenkorthese bei der Bewegung und Streckung möglich ist.

Aus Arbeiten aus der Kniegelenkbiomechanik ist wissenschaftlich erwiesen, daß das menschliche Kniegelenk einer mehrdimensionalen Bewegung gehorcht.
Beugung und Streckung- des Gelenks erfolgen um eine Drehachse, die nicht fest lokalisiert ist und die somit ihre Lage ändert Dies ergibt sich aus der Lage des vorderen und hinteren Kreuzbandes zueinander Wissenschaftliche Untersuchungen haben gezeigt, daß sich der Drehpunkt während der Beugung auf der Gelenkaußenseite um ungefähr 14 mm nach dorsal und ungefähr 3 mm nach caudal verlagert. Die Lageveränderung der Drehachse auf der Gelenkinnenseite ist vernachlässigbar klein. Hieraus resultiert eine Auswärtsdrehung des Unterschenkels während der Beugung.

Zur Protektion des verletzten und gegebenenfalls operativ rekonstruierten Kniegelenks werden heute sogenannte funktionelle Orthesen eingesetzt. Grundprinzip ihrer Konstruktion ist ein fester Rahmen, bestehend aus einem Oberschenkel- und einem Unterschenkeln. Beide Teile dieser Orthese sind durch ein Gelenk auf der Medial- und der Lateralseite miteinander verbunden.

US-A-4 966 133 beschreibt eine Kniegelenkorthese mit einem Unterschenkelteil, einem Oberschenkelteil sowie einem lateralen und einem medialen Orthesengelenk, die das Unterschenkelteil und das Oberschenkelteil miteinander verbinden. Das laterale Orthesengelenk wird von einem Gelenk, das aus zwei ineinandergreifenden Zahnrädern, deren Achren beidseitig mit jeweils einer Gelenkplatte fest verbunden sind, gebildet.

Jedes der beiden Zahnräder weist jeweils eine Strebe auf, die zur festen Verbindung des lateralen Orthesengelenks mit dem Unterschenkel bzw. dem Oberschenkelteil dienen. Das mediale Orthesengelenk wird von einem einachsigen Gelenk gebildet.

Bei den bisherigen Produkten, die im Stand der Technik bekannt sind, sind die Gelenke auf beiden Seiten baugleich, seien sie einachsig oder polyzentrisch.
Daraus resultiert ausnahmslos eine Mechanik, die einer Drehachse gehorcht. Einige Konstruktionen verlagern die Drehachse symmetrisch nach hinten, d.h., der Drehpunkt wandert gleichmäßig auf der Außen- und Innenseite nach hinten aus. Die aus biomechanischen Gesichtspunkten erforderliche Unterschenkelrotation wird nicht erlaubt.

Aufgabe der Erfindung ist es, eine Kniegelenkorthese zu schaffen, die wie das menschliche Kniegelenk in der Lage ist, bei der Bewegung und Streckung des Gelenks die auftretende Verlagerung der Drehachse zu ermöglichen.

Gelöst wird diese Aufgabe durch die Kniegelenkorthese, wie sie in Anspruch 1 gekennzeichnet ist.

Vorteilhafte Ausgestaltungen der Kniegelenkorthese sind dabei Gegenstand der Unteransprüche.

Die erfindungsgemäße Kniegelenkorthese besteht aus einem Unterschenkelteil und einem Oberschenkelteit, die an dem jeweiligen Beinabschnitt fixiert werden.
Die Verbindung des Unterschenkelteils und des Oberschenkelteils erfolgt durch ein laterales und ein mediales Orthesengelenk.
Das laterale Orthesengelenk ist von einem Gelenk gebildet, das aus zwei ineinandergreifenden Zahnrädern besteht, deren Achsen beidseitig mit jeweils einer Gedenkplatte fest verbunden sind. Jedes der beiden Zahnräder weist jeweils eine Strebe auf, die die Verbindung des lateralen Orthesengelenks mit dem Unterschenkelteil und dem Oberschenkelteil sicherstellt.
Das mediale Orthesengelenk ist von einem einachsigen Gelenk gebildet, das sich aus einem U-förmigen Oberteil und einem von dem Oberteil umfaßten Unterteil zusammensetzt, wobei Oberteil und Unterteil gelenkig durch einen Zapfen miteinander verbunden sind. Vorteilhafterweise weist der Zapfen im gelenkbildenden Abschnitt eine leichte Kugelform auf.

Die Verbindung des Unterschenkelteils bzw. des Oberschenkelteils mit dem lateralen und dem medialen Orthesengelenk erfolgt vorzugsweise mittels einer Justiervorrichtung.
Zur Bildung der Justiervorrichtung weisen die obere und die untere Strebe des lateralen Orthesengelenks, das Oberteil und das Unterteil des medialen Orthesengelenks sowie der distale Abschnitt des Oberschenkelteils in der lateralen und in der medialen Seite bzw. der proximale Abschnitt des Unterschenkelteils in der lateralen und in der medialen Seite jeweils ein Langloch auf. Die Langlöcher in dem Oberschenkelteil und dem Unterschenkelteil sind gegenüber den Langlöchern in den Streben des lateralen Orthesengelenks bzw. in dem Oberteil und in dem Unterteil des medialen Orthesengelenks jeweils um insbesondere 90° versetzt. Die jeweils beiden korrespondierenden Langlöcher sind zentrisch über einen Gleitbock verknüpft, wobei mit Hilfe einer zentralen Feststellschraube eine Bewegung der jeweiligen Teile verhindert wird.

Das obere der beiden Zahnräder in dem lateralen Orthesengelenk einen kleineren Radius auf als das untere, wobei das obere vorzugsweise einen Radius von 15mm und das untere vorzugsweise einen Radius von 27 mm aufweist.
Somit ist die vorteilhafte Verlagerung des Drehpunkts des lateralen Orthesengelenks während der Bewegung der Kniegelenkorthese von 0° Streckung zu 90° Bewegung von ungefähr 14 mm nach dorsal und 3 mm nach caudal möglich.

Zur Begrenzung der Extension und der Flexion der Kniegelenkorthese sind an dem lateralen Orthesengelenk und an dem medialen Orthesengelenk vorteilhafterweise Anschlagmittel vorgesehen.

Beim lateralen Orthesengelenk besteht die Vorrichtung zur Begrenzung der Extension und der Flexion vorzugsweise aus einem Langloch in Form eines Kreisabschnitts in dem unteren Zahnrad, aus Langlöchern ebenfalls in Form eines Kreisabschnitts auf der medialen Gelenkplatte und der lateralen Gelenkplatte, wobei bei gestreckter Kniegelenkorthese die drei Langlöcher kongruent sind. Die Langlöcher auf den Gelenkplatten weisen vorzugsweise eine etwas geringe Breite auf als das Langloch in dem unteren Zahnrad.
Desweiteren finden zwei Hülsen Verwendung, die sich von der medialen Gelenkplatte bis ins Langloch der lateralen Gelenkplatte erstrecken. Im Inneren der Hülsen sind Schrauben geführt, die mittels Muttern, die in dem Langloch der medialen Gelenkplatte geführt werden und somit gegen ein Vedrehen gesichert sind. die Hülsen in ihrer Stellung in dem Langloch auf der lateralen Gelenkplatte fixieren.

Das Oberschenkelteil und das Unterschenkelteil der Kniegelenkorthese bestehen vorzugsweise von einer Rahmenkonstruktion, die insbesondere ventral am Bein angelegt wird. Diese Rahmenkonstruktion wird dabei vorzugsweise in Sandwichbauweise aus einer Komposition aus einem individuell anformbaren Leichtmetallrahmen mit entsprechenden Innenpolstern und einer äußeren Rahmenbeschichtung aufgebaut.

Die erfindungsgemäße Kniegelenkorthese bietet ein Reihe von Vorteilen, die die Verwendung dieser bei einer notwendigen Ruhigstellung oder postoperativen Rehabilitation des Knies erforderlich macht.
So ist durch die Verwendung zweier unterschiedlich ausgeformter Orthesengelenke, von denen das laterale Orthesengelenk ein Zahnradgelenk ist, die Verschiebung der Drehachse der Kniegelenkorthese möglich, wie es nach den neusten wissenschaftlichen Erkenntnissen notwendig ist, um den natürlichen Bewegungsablauf des Kniegelenks nicht zu beeinträchtigen.

Durch die besondere Ausführungsform des lateralen Orthesengelenks ist darüber hinaus die notwendige Limitierung der Extension und der Flexion der Kniegelenkorthese besonders bequem einzustellen.
Die vorteilhafte Ausführung des medialen Orthesengelenks als eine Art Kugelgelenk erlaubt die dreidimensionale Bewegung des medialen Orthesengelenks, womit ein Verklemmen desselben bei der Wanderung der Drehachse während eines Bewegungsvorganges ausgeschlossen ist. Auch dieses Orthesengelenk ermöglicht auf besonders einfache Art und Weise die Extension und die Flexion der Kniegelenkorthese zu begrenzen.
Die besondere Justiervorrichtung, die zur Verbindung der beiden Orthesengelenke mit dem Oberschenkel- bzw. Unterschenkelteil dient, ist es möglich, die Kniegelenkorthese individuell in die anatomisch optimale Position zu bringen und gleichzeitig Mikrobewegungen gegenüber dem Kniegelenk auf ein Mindestmaß zu reduzieren zudem kann durch diese Justiervorrichtung eine Varus- oder Valguskorrektur vorgenommen werden.
Die Rahmenkonstruktion des Ober- und Unterschenkelteil in Sandwichbauweise in einer Komposition aus einem individuell anformbaren Leichtmetallrahmen mit entsprechenden Innenpolstern und einer äußeren Rahmenbeschichtung schützt das Kniegelenk besonders vorteilhaft gegen äußere Schlag- und Stoßeinwirkungen, gleichzeitig wird aber ein vollständiges Beugen des Kniegelenks durch die ventrale Lage am Bein nicht behindert
Darüber hinaus bietet die Rahmenkonstruktion den Vorteil, die Kleidung gegen mechanische Einflüsse zu schützen.

Ein Ausführungsbeispiel ist ein den Zeichnungen dargestellt. Im einzelnen zeigen:
- Figur 1: die Kniegelenkorthese für das rechte Bein, gesehen von der lateralen Seite,
- Figur 2: das laterale Orthesengelenk, und zwar die laterale Seite,
- Figur 3: das laterale Orthesengelenk ohne laterale Gelenkplatte,
- Figur 4: das laterale Orthesengelenk im senkrechten Schnitt gemäß Linie A-A in Figur 3,
- Figur 5: das mediale Orthesengelenk, und zwar die laterale Seite,
- Figur 6: das Oberteil des medialen Orthesengelenks von medial,
- Figur 7: das Unterteil des medialen Orthesengelenks
- Figur 8: das mediale Orthesengelenk in senkrechtem Schnitt gemäß Linie B-B in Figur 5,
- Figur 9: die einzelnen Bestandteile der Fixierung der Justiervorrich tung,
- Figur 10: eine Fixierung der Justiervorrichtung, und zwar von lateral und von anterior.

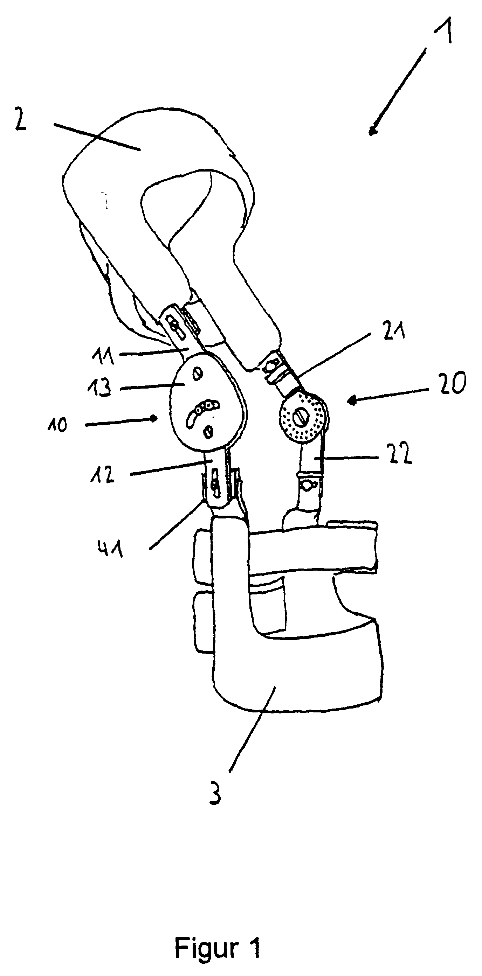

In Figur 1 ist eine besonders vorteilhafte Ausgestaltung der Kniegelenkorthese (1) dargestellt, und zwar einer Kniegelenkorthese (1) für das rechte Bein. Die Kniegelenkorthese (1) besteht aus einem Oberschenkelteil (2), einem Unterschenkelteil (3), einem lateralen Orthesengelenk (10), bestehend aus einem oberen Zahnrad (11), einem unteren Zahnrad (12) und einer lateralen Gelenkplatte (13), sowie einem medialen Orthesengelenk (20), bestehend aus einem Oberteil (21) und einem Unterteil (22), die gelenkig miteinander verbunden sind. Die Verbindung von Oberschenkelteil (2) und Unterschenkelteil (3) mit den beiden Orthesengelenken (10, 20) erfolgt jeweils über eine sogenannte Fixierung, deren entscheidendes Bauteil jeweils ein Gleitbock (41) darstellt.

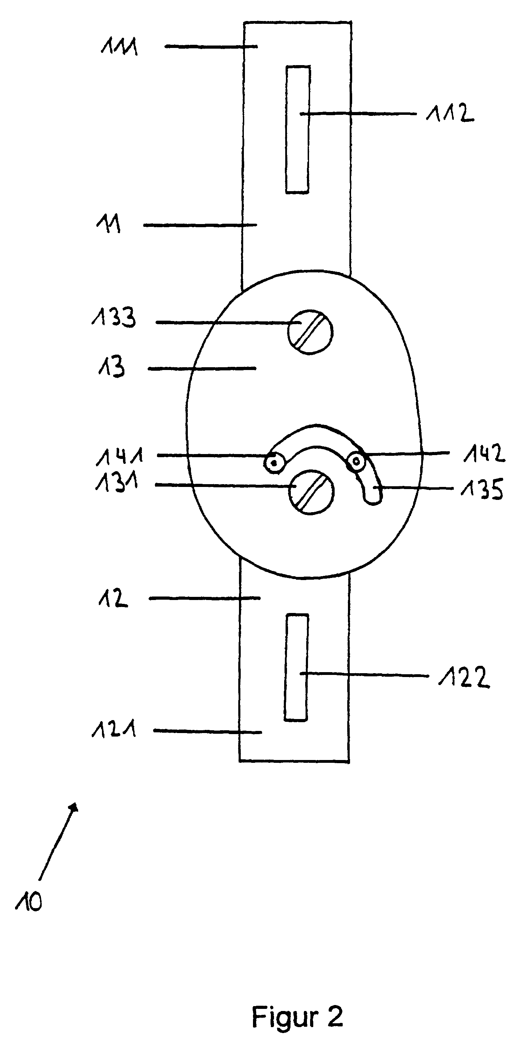

Gemäß Figur 2 wird das laterale Orthesengelenk (10) von dem oberen Zahnrad (11) und dem unteren Zahnrad (12) gebildet. Zur Verbindung dient die laterale Gelenkplatte (13), die mittels von Schrauben (131, 133) die Zahnräder (11, 12) fixiert. Sowohl die Strebe (111) des oberen Zahnrads (11) als auch die Strebe (121) des unteren Zahnrads (12) weisen ein Langloch (112, 122) auf, das zur Verbindung mit dem Oberschenkelteil (2) bzw. dem Unterschenkelteil (3) der Kniegelenkorthese (1) dient. Die laterale Gelenkplatte (13) ist bevorzugt von ellipsenförmiger Gestalt, um die Zahnkränze der Zahnräder (11, 12) sicher abzudecken.
Weiterhin weist die laterale Gelenkplatte (13) ein Langloch (135) in Form eines Kreisabschnitts auf, das zur Aufgabe hat, die maximale Extension und Flexion durch die beiden festgesetzten Hülsen (141, 142) zu begrenzen.

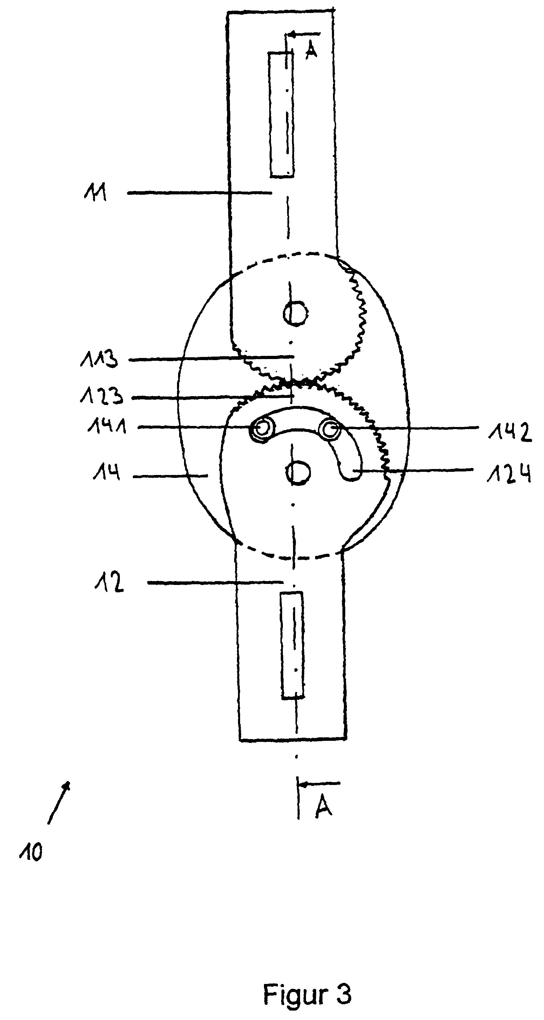

In Figur 3 ist das laterale Orthesengelenk (10) nach Abnahme der lateralen Gelenkplatte (13) dargestellt. Die beiden Zahnräder (11, 12) sind fest mit der medialen Gelenkplatte (14), die vorzugsweise die gleichen Abmessungen wie die laterale Gelenkplatte (13) aufweist, verbunden. Nahe des Flächenschwerpunkts der medialen Gelenkplatte (14) greifen die Zahnkränze (113, 123) der beiden Zahnräder (11, 12) ineinander.
Das untere Zahnrad (12) weist eine Vorrichtung zur Begrenzung der maximalen Extension und Flexion des lateralen Orthesengelenks (10) auf. Die genannte Vorrichtung setzt sich aus einem Langloch (124) in Form eines Kreisausschnitts in dem unteren Zahnrad (12), bei gestrecktem Orthesengelenk (10) einem zum Langloch (124) kongruenten Langloch in Form eines Kreisabschnitts auf der medialen Gelenkplatte (14), dem ebenfalls kongruenten Langloch (135) auf der lateralen Gelenkplatte (13) sowie zwei Hülsen (141, 142) zusammen, die mittels von Schrauben und Muttern fixierbar sind und die sich bis in das Langloch (135) in der lateralen Gelenkplatte (13) erstrecken. Die anterior liegende Hülse (141) begrenzt dabei die Extension des lateralen Orthesengelenks (10), die posterior gelegene Hülse (142) die Flexion.

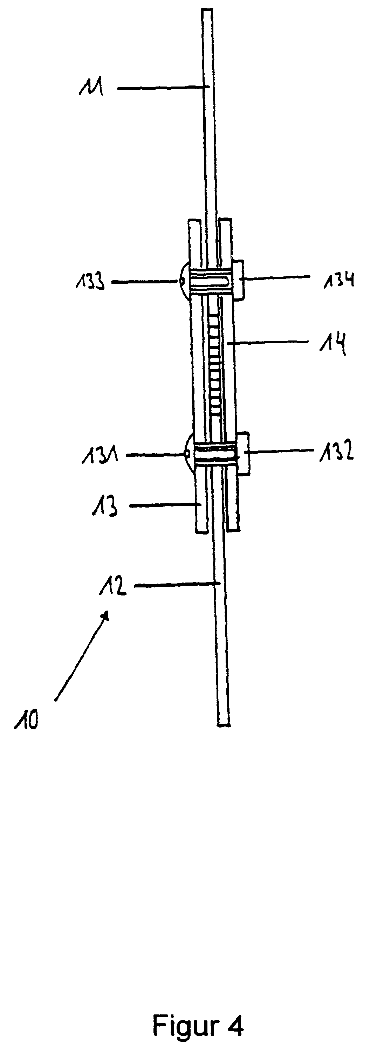

Die Figur 4 zeigt das laterale Orthesengelenk (10) in einem senkrechten Schnitt gemäß Linie A-A in Figur 3. Zwei Schrauben (131, 133) fixieren die laterale Gelenkplatte (13), die zu dieser (13) kongruent liegende mediale Gelenkplatte (14) sowie das obere Zahnrad (11) und das untere Zahnrad (12). Gekontert werden die Schrauben (131, 133) von zwei Muttern (132, 134).

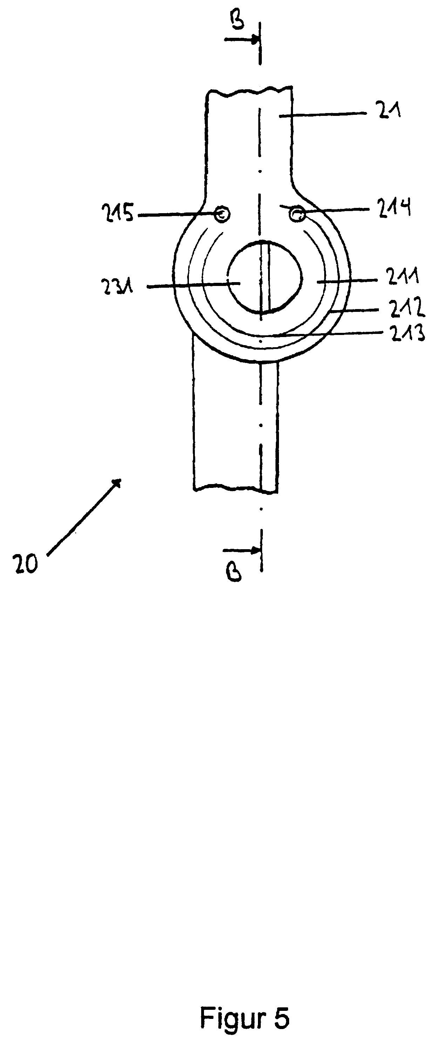

Die Figur 5 zeigt das mediale Orthesengelenk (20) von der lateralen Seite. Das Oberteil (21) weist einen bevorzugt kreisförmigen lateralen Endabschnitt (211) auf. Auf diesem Endabschnitt (211) sind zwei konzentrische Kreise (212, 213) aus äquidistanten Bohrungen vorgesehen, dessen Mittelpunkt die Gelenkachse des medialen Orthesengelenks (20) darstellt. Mit Hilfe von zwei Feststellschrauben (214, 215) kann nun die Extension und Flexion des medialen Orthesengelenks (20) limitiert werden.
Eine in der Gelenkachse befindliche Schraube (231) stellt die Verbindung von Oberteil (21) und Unterteil (22) sicher.

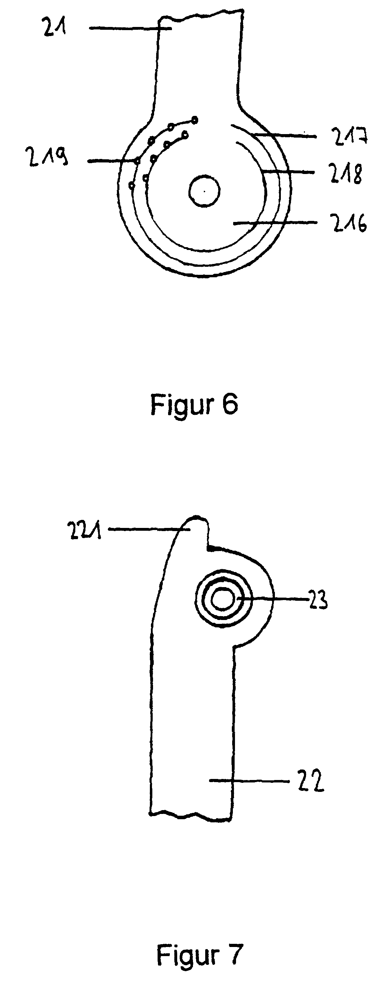

Die Figur 6 stellt das Oberteil (21) des medialen Orthesengelenks (20) dar, und zwar von der medialen Seite. Auf den wiederum kreisförmigen medialen Endabschnitt (216) sind zwei konzentrische Kreise (217, 218) aus äquidistanten Gewindebohrungen (219). Diese Kreise (217, 218) sind kongruent zu den Kreisen (212, 213) auf dem lateralen Endabschnitt (211) angeordnet. Im Zusammenspiel mit den Feststellschrauben (214, 215) kann somit die Extension und die Flexion des medialen Orthesengelenks (20) in bevorzugt 10°-Schritten limitiert werden.

Wie in Figur 7 gezeigt, ist mit dem Unterteil (22) der bevorzugt eine leichte Kugelform aufweisende Zapfen (23) fest eingepreßt. Die Nase (221) liegt zwischen den Feststellschrauben (214, 215) und begrenzt auf diese Weise die Bewegungsmöglichkeit des medialen Orthesengelenks (20).

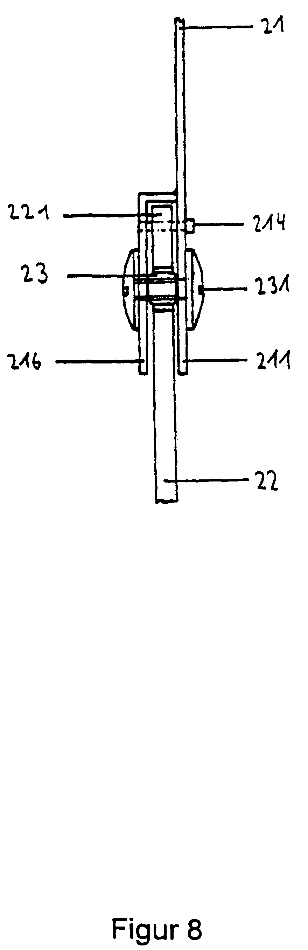

In Figur 8 ist das mediafe Orthesengelenk in senkrechtem Schnitt gemäß Linie B-B in Figur 5 dargestellt., das U-förmige Oberteil (21) umfaßt mit den beiden bevorzugt kreisförmigen Endabschnitten (211, 216) das Unterteil (22). in der Gelenkachse befindet sich die Schraube (231), die durch den Zapfen (23) gehend die sichere Verbindung von Oberteil (21) und Unterteil (22) sicherstellt. Die an dem Unterteil (22) vorgesehene Nase (221) erstreckt sich bis in den Bereich des Oberteils (21), in dem die Feststellschrauben (214, 215) angeordnet sind.

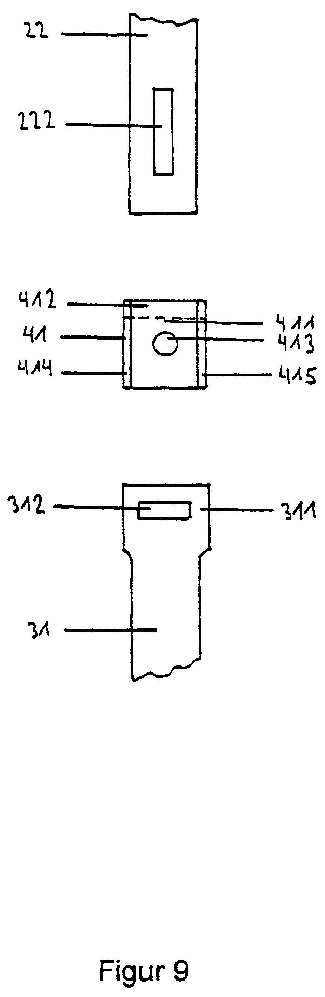

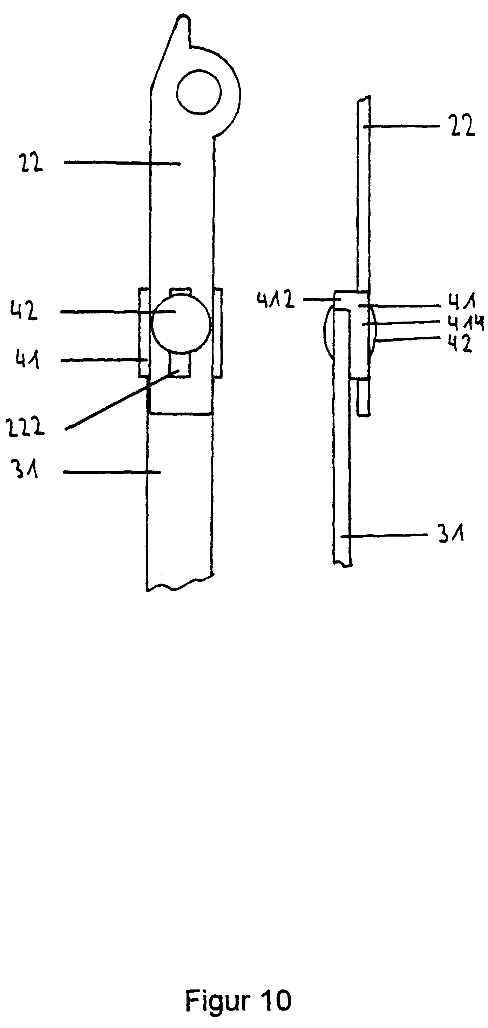

Beispielhaft für die vier vorhandenen Fixierungen der Justiervorrichtung der Kniegelenkorthese (1) zeigen die Figuren 9 und 10 die Fixierung, die die Verbindung des Unterteils (22) des medialen Orthesengelenks (20) mit dem Unterschenkelteil (31) der Kniegelenkorthese sicherstellt, und zwar einmal von lateral und zum anderen von anterior im montierten Zustand und die einzelnen Bauteile. Die Fixierung besteht gemäß Figur 9 aus Unterteil (22), Gleitbock (41) und proximalem Abschnitt des Unterschenkelteils (31). In dem Unterteil (22) ist in distaler Richtung ein Langloch (222) vorgesehen. Dementsprechend weist der proximale Abschnitt des Unterschenkelteils (31) an seinem rechteckigen Endstück (311) ein Langloch (312) in anteriorer Richtung auf.
Die Verbindung der beiden somit um 90° versetzten Langlöcher (222, 312) erfolgt durch einen Gleitbock (41). Der Gleitbock (41) hat in medialer Richtung eine U-förmige Vertiefung (411), die zur Aufnahme des Unterteils (22) dient und die somit eine Bewegung durch die seitlichen Kanten (414, 415) des Unterteils (22) nach anterior oder posterior ausschließt. Weiterhin besitzt der Gleitbock (41) zentral eine Bohrung (413), die mit den Langlöchern (222, 312) korrespondiert. Um eine Bewegung des proximalen Abschnitts des Unterschenkelteils (31) nach proximal zu verhindern, weist der Gleitbock (41) eine über seine gesamte Breite erstreckende Anschlagkante (412) auf.

Die Figur 10 zeigt die Fixierung im montierten Zustand. Die Langlöcher (222, 312) erlauben in Verbindung mit dem Gleitbock (41) die Fixierung individuell an die gegebenen Maße von Knie und bei dieser Fixierung Unterschenkel des Trägers der Kniegelenkorthese (1) anzupassen. Die Arretierung erfolgt mittels einer Schraube (42).

Dementsprechend sind auch an den anderen drei Verknüpfungspunkten der Orthesengelenke (10, 20) mit dem Oberschenkelteil (2) und dem Unterschenkelteil (3) Fixierungen vorgesehen, die die optimale Anpassung der Kniegelenkorthese (1) an die physischen Gegebenheiten beim betroffenen Knie des Trägers ermöglichen.

## Patentansprüche

1. Kniegelenkorthese mit einem Unterschenkelteil (3), einem Oberschenkelteil (2) sowie einem lateralen (10) und einem medialen Orthesengelenk (20), die das Unterschenkelteil (3) und das Oberschenkelteil (2) miteinander verbinden, wobei das laterale Orthesengelenk (10) aus zwei ineinandergreifenden Zahnrädern (11, 12), deren Achsen beidseitig mit jeweils einer Gelenkplatte (13, 14) fest verbunden sind, besteht, jedes der beiden Zahnräder (11, 12) jeweils eine Strebe (111, 121) aufweist, wobei zur festen Verbindung des lateralen Orthesengelenks (10) mit dem Unterschenkelteil (3) die Strebe (121), und zur Verbindung mit dem Oberschenkelteil (2) die Strebe (111) dient, und das mediale Orthesengelenk (20) von einem einachsigen Gelenk gebildet ist, **dadurch gekennzeichnet, dass**
das mediale Orthesengelenk (20) aus einem U-förmigen Oberteil (21), das ein Unterteil (22) umfaßt, wobei Oberteil (21) und Unterteil (22) gelenkig durch einen Zapfen (23) miteinander verbunden sind, gebildet wird und das obere der beiden Zahnräder (11) in dem lateralen Orthesengelenk (10) einen kleineren Radius aufweist als das untere Zahnrad (12).

2. Kniegelenkorthese nach Anspruch 1, **dadurch gekennzeichnet, dass** das obere Zahnrad (11) einen Radius von 15 mm und das untere Zahnrad (12) einen Radius von 27 mm aufweist.

3. Kniegelenkorthese nach Anspruch 1, **dadurch gekennzeichnet, dass** der Zapfen (23) des medialen Orthesengelenks (20) im gelenkbildenden Abschnitt eine leichte Kugelform aufweist.

4. Kniegelenkorthese nach den Ansprüchen 1 bis 3, **dadurch gekennzeichnet, dass** die Verbindung des Unterschenkelteils (3) mit dem lateralen (10) und dem medialen (20) Orthesengelenk bzw. des Oberschenkelteils (2) mit dem lateralen (10) und medialen (20) Orthesengelenk jeweils einer Justiervorrichtung erfolgt, gebildet von
jeweils einem Langloch (112, 122) in der oberen (111) und unteren (121) Strebe des lateralen Orthesengelenks (10), jeweils einem Langloch in dem Oberteil (21) und in dem Unterteil (22) des medialen Orthesengelenks,
jeweils einem Langloch in der lateralen und in der medialen Seite auf dem distalen Abschnitt des Oberschenkelteils,
jeweils einem Langloch in der lateralen und in der medialen Seite auf dem proximalen Abschnitt des Oberschenkelteils,
jeweils einem Langloch in der lateralen und in der medialen Seite auf dem proximalen Abschnitt des Unterschenkelteils (31),
wobei die Langlöcher in dem Oberschenkel- und dem Unterschenkelteil gegenüber den Langlöchern in den Streben bzw. in dem Oberteil (21) und in dem Unterteil (22) jeweils insbesondere um 90° versetzt sind, und
wobei jeweils die beiden korrespondierenden Langlöcher zentrisch über einen Gleitbock (41) mit einer Feststellschraube (42) verbunden sind.

5. Kniegelenkorthese nach den Ansprüchen 1 bis 4, **dadurch gekennzeichnet, dass** die Extension und die Flexion des lateralen Orthesengelenks und des medialen Orthesengelenks durch Anschlagmittel (412) einschränkbar ist.

6. Kniegelenkorthese nach den Ansprüchen 1 bis 5, **dadurch gekennzeichnet, dass** die Vorrichtung zur Begrenzung der Extension und der Flexion beim lateralen Orthesengelenk besteht aus
einem Langloch (124) in Form eines Kreisabschnitts in dem unteren Zahnrad (12),
einem Langloch in Form eines Kreisabschnitts in der medialen Gelenkplatte (14),
einem Langloch (135) in Form eines Kreisabschnitts in der lateralen Gelenkplatt (13),
wobei bei gestreckter Kniegelenkorthese die drei Langlöcher kongruent sind, sowie zwei Hülse (141, 142), die
a) sich von der medialen Gelenkplatt (14) bis in das Langloch (135) der lateralen Gelenkplatte (13) ersfrecken,
b) mittels Schrauben, die im Inneren der Hülsen geführt werden, und Muttern, die im Langloch der medialen Gelenkplatte (14) geführt werden, in ihrer Stellung im Langloch (135) der lateralen Gelenkplatte (13) fixlerbar sind.

7. Kniegelenkorthese nach den Ansprüchen 1 bis 6, **dadurch gekennzeichnet, dass** das Oberschenkelteil (2) und das Unterschenkelteil (3) aus einer Rahmenkonstruktion bestehen.

8. Kniegelenkorthese nach Anspruch 7, **dadurch gekennzeichnet, dass** die Rahmenkonstruktion in Sandwichbauweise aus einer Komposition aus einem individuell anformbaren Leichtmetallrahmen mit entsprechenden Innenpolstern und einer äußeren Rahmenbeschichtung besteht.

## Claims

1. Knee-joint orthesis with a lower leg part (3) and a thigh part (2), and with a lateral orthesis hinge (10) and a medial orthesis hinge (20) which connect the lower leg part (3) and the thigh part (2) to each other, wherein the lateral orthesis hinge (10) consists of two intermeshing toothed wheels (11, 12) whose axles are each securely connected on both sides to a hinge plate (13, 14), each of the two toothed wheels (11, 12) having in each case a strut (111, 121), strut (121) being used for securely connecting the lateral orthesis hinge (10) to the lower leg part (3) and strut (111) being used for connection to the thigh part (2), and the medial orthesis hinge (20) is formed by a single-axle hinge, **characterized in that** the medial orthesis hinge (20) is formed from a U-shaped upper part (21) which encloses a lower part (22), the upper part (21) and lower part (22) being connected to each other in an articulated manner by means of a pin (23), and the upper of the two toothed wheels (11) in the lateral orthesis hinge (10) has a smaller radius than the lower toothed wheel (12).

2. Knee-joint orthesis according to Claim 1, **characterized in that** the upper toothed wheel (11) has a radius of 15 mm and the lower toothed wheel (12) has a radius of 27 mm.

3. Knee-joint orthesis according to Claim 1, **characterized in that** the pin (23) of the medial orthesis hinge (20) has a slight spherical shape in the hinge-forming section.

4. Knee-joint orthesis according to Claims 1 to 3, **characterized in that** the connection of the lower leg part (3) to the lateral orthesis hinge (10) and the medial orthesis hinge (20), and of the thigh part (2) to the lateral orthesis hinge (10) and the medial orthesis hinge (20), is effected in each case using an adjustment device, formed by in each case an oblong slot (112, 122) in the upper strut (111) and lower strut (121) of the lateral orthesis hinge (10),
in each case an oblong slot in the upper part (21) and in the lower part (22) of the medial orthesis hinge, in each case an oblong slot in the lateral side and in the medial side on the distal section of the thigh part,
in each case an oblong slot in the lateral side and in the medial side on the proximal section of the thight part,
in each case an oblong slot in the lateral side and in the medial side on the proximal section of the lower leg part (31),
where the oblong slots in the thigh part and the lower leg part are in each case offset in particular by 90° in relation to the oblong slots in the struts and in the upper part (21) and the lower part (22), and
where the two corresponding oblong slots are in each case connected centrally via a slide bracket (41), using a locking screw (42).

5. Knee-joint orthesis according to Claims 1 to 4, **characterized in that** the extension and flexion of the lateral orthesis hinge and of the medial orthesis hinge can be limited by stop means (412).

6. Knee-joint orthesis according to Claims 1 to 5, **characterized in that** the device for limiting the extension and flexion on the lateral orthesis hinge consists of
an oblong slot (124) in the form of a portion of a circle in the lower toothed wheel (12),
an oblong slot in the form of a portion of a circle in the medial hinge plate (14),
an oblong slot (135) in the form of a portion of a circle in the lateral hinge plate (13),
where the three oblong slots are congruent when the knee-joint orthesis is extended, and
two sleeves (141, 142) which
a) extend from the medial hinge plate (14) into the oblong slot (135) of the lateral hinge plate (13),
b) can be fixed in their position in the oblong slot (135) of the lateral hinge plate (13) by means of screws, which are guided in the inside of the sleeves, and nuts, which are guided in the oblong slot of the medial hinge plate (14).

7. Knee-joint orthesis according to Claims 1 to 6, **characterized in that** the thigh part (2) and the lower leg part (3) consist of a frame structure.

8. Knee-joint orthesis according to Claim 7, **characterized in that** the frame structure, of sandwich design, consists of a combination of an individually shapeable light metal frame with corresponding inner pads and of an outer frame covering.

## Revendications

1. Orthèse d'articulation de genou comportant une pièce de bas de cuisse (3), une pièce de haut de cuisse (2) ainsi qu'une articulation d'orthèse latérale (10) et médiale (20) qui relie l'une à l'autre la pièce de bas de cuisse (3) et la pièce de haut de cuisse (2), l'articulation d'orthèse latérale (10) étant composée de deux engrenages (11, 12) s'engrenant l'un dans l'autre dont les axes sont reliés fixement des deux côtés chacun à une plaque d'articulation (13, 14), chacun des deux engrenages (11, 12) présentant respectivement une traverse (111, 121), la traverse (121) servant à relier fixement l'articulation d'orthèse latérale (10) à la pièce de bas de cuisse (3) et la traverse (111) à la relier à la pièce de haut de cuisse (2) et l'articulation d'orthèse médiale (20) étant constituée par une articulation à axe unique, **caractérisée en ce que** l'articulation d'orthèse médiale (20) est constituée d'une pièce supérieure en forme de U (21) qui comprend une pièce inférieure (22), la pièce supérieure (21) et la pièce inférieure (22) étant reliées l'une à l'autre de manière articulée par un tourillon (23) et que l'engrenage supérieur parmi les deux engrenages (11) présente dans l'articulation d'orthèse latérale (10) un rayon plus petit que l'engrenage inférieur (12).

2. Orthèse d'articulation de genou selon la revendication 1, **caractérisée en ce que** l'engrenage supérieur (11) présente un rayon de 15 mm et l'engrenage inférieur (12) un rayon de 27 mm.

3. Orthèse d'articulation de genou selon la revendication 1, **caractérisée en ce que** le tourillon (23) de l'articulation d'orthèse médiale (20) présente une légère forme sphérique dans la section formant l'articulation.

4. Orthèse d'articulation de genou selon les revendications 1 à 3, **caractérisée en ce qu'**il est établi la liaison de la pièce de bas de cuisse (3) avec l'articulation d'orthèse latérale (10) et médiale (20) ou de la pièce de haut de cuisse (2) avec l'articulation d'orthèse latérale (10) et médiale (20) de respectivement un dispositif d'ajustage, constituée par
respectivement un trou oblong (112, 122) dans la traverse supérieure (111) et inférieure (121) de l'articulation d'orthèse latérale (10),
respectivement un trou oblong dans la pièce supérieure (21) et dans la pièce inférieure (22) de l'articulation d'orthèse médiale,
respectivement un trou oblong dans la face latérale et médiale sur la section distale de la pièce de haut de cuisse,
respectivement un trou oblong dans la face latérale et médiale sur la section proximale de la pièce de haut de cuisse,
respectivement un trou oblong dans la face latérale et médiale sur la section proximale de la pièce de bas de cuisse (31),
les trous oblongs dans la pièce de haut de cuisse et de bas de cuisse étant décalés chacun notamment de 90° par rapport aux trous oblongs pratiqués dans les traverses ou dans la pièce supérieure (21) et dans la pièce inférieure (22), et
les deux trous oblongs respectivement correspondants étant reliés de manière centrée par un chevalet coulissant (41) à une vis de fixation (42).

5. Orthèse d'articulation de genou selon les revendications 1 à 4, **caractérisée en ce que** l'extension et la flexion de l'articulation d'orthèse latérale et de l'articulation d'orthèse médiale peuvent être limitées par des moyens de butée (412).

6. Orthèse d'articulation de genou selon les revendications 1 à 5, **caractérisée en ce que** le dispositif de limitation de l'extension et de la flexion dans l'articulation d'orthèse latérale consiste en
un trou oblong (124) sous forme d'un segment de cercle dans l'engrenage inférieur (12),
un trou oblong sous forme d'un segment de cercle dans la plaque d'articulation médiale (14),
un trou oblong (135) sous forme d'un segment de cercle dans la plaque d'articulation latérale (13),
les trois trous oblongs étant congruents lors de l'extension de l'orthèse d'articulation de genou, ainsi que deux manchons (141, 142) qui
a) s'étendent de la plaque d'articulation médiale (14) jusque dans le trou oblong (135) de la plaque d'articulation latérale (13),
b) peuvent être fixés dans leur position dans le trou oblong (135) de la plaque d'articulation latérale (13) au moyen de vis qui sont guidées à l'intérieur des manchons et d'écrous qui sont guidés dans le trou oblong de la plaque d'articulation médiale (14).

7. Orthèse d'articulation de genou selon les revendications 1 à 6, **caractérisée en ce que** la pièce de haut de cuisse (2) et la pièce de bas de cuisse (3) consistent en une construction en forme de cadre.

8. Orthèse d'articulation de genou selon la revendication 7, **caractérisée en ce que** la construction en forme de cadre consiste, sous forme d'une construction en sandwich, en une composition d'un cadre en métal léger façonnable individuellement comportant des coussins intérieurs appropriés et d'un revêtement de cadre extérieur.
